# EUROPEAN PATENT APPLICATION

(11) **EP 4 316 353 A1**
(43) Date of publication of application: **07.02.2024**
(21) Application number: 23190003.6
(22) Date of filing: 07.08.2023
(51) Int. Cl.: A61B 3/113, A61B 5/18

(54) **DEVICE FOR A SURGICAL IMAGING SYSTEM, SURGICAL IMAGING SYSTEM, METHOD AND COMPUTER PROGRAM**

(30) Priority: 05.08.2022 DE 102022119783
(71) Applicant: Leica Instruments (Singapore) Pte Ltd, Singapore 608924 (SG)
(72) Inventor: TILLE, Sebastian, 608924 Singapore (SG)
(74) Representative: 2SPL Patentanwälte PartG mbB

(57) **Abstract**

A device 130 for a surgical imaging system is provided. The device 130 comprises interface circuitry 132 configured to receive, from an eye tracking sensor, an output comprising information indicative of a status of an eye of a user viewing a display and processing circuitry 134 configured to determine a condition of the user based on the received output signal.

## Description

### Technical field

Examples relate to device for a surgical imaging system, a surgical imaging system, a method and a computer program.

### Background

Eye tracking, which enables determination of eye positions or eye movements, has emerged as an important tool for many applications, e.g., imaging systems used for microsurgery. Eye tracking, which provides real time eye positions, can be very important for the performance of extended reality systems, such as Augmented Reality (AR) systems and Virtual Reality (VR) systems. Eye position values, such as the distance between the eyes and an exact distance between the eyes and an AR/VR display, can be important for calibration of head-mounted display systems, in order provide optical sharpness and high image quality. Knowing eye positions is also key for advanced display techniques such as foveated rendering, which involves rendering portions of images that are gazed by the fovea in high detail, while reducing the image quality in the peripheral vision. In principle, eye tracking technology determines the gaze position based on a calibrated system that assigns determined viewing angles with corresponding positions on a defined viewing area. However, especially in the field of microsurgery a detection of the gaze position may not cover multiple scenarios. Thus, there may be a need to improve a use case of eye tracking for a surgical imaging system.

### Summary

It is a finding that a determination of a user behavior and/or a cognitive process of a user using an imaging system can be increased by determining a condition of the user. The user condition may be determined based on an output signal of an eye tracking sensor, allowing e.g., to determine a fatigue of the user. This way, information derived from the use of an eye tracking sensor can be increased.

Examples provide a device for an imaging system comprising interface circuitry configured to receive from an eye tracking sensor an output signal comprising information indicative of a status of an eye of a user viewing a display and processing circuitry configured to determine a condition of the user based on the received output signal. This way, a human behavior and/or a cognitive process of user can be determined. Thus, a user can be informed about a determined condition which may negatively influence a process performed by the user.

In an example, the interface circuitry may be further configured to receive information about a position of the display. Further, the processing circuitry may be configured to determine a focus point of the eye of the user and to determine the condition of the user based on the focus point of the eye of the user and the position of the display.

In an example, the information about the user viewing the display may correspond to a first eye of the user. Further, the interface circuitry may be configured to receive, from a further eye tracking sensor, a second output signal comprising information indicative of a status of a second eye of the user viewing the display. Further, the processing circuitry may be configured to determine a condition of the user based on the received output signal and the second output signal for the first eye and the second eye. This way, the information about both eyes of a user can be combined to determine a status of the user, e.g., by comparing the movement of both eyes of the user.

In an example, the condition of the user may be determined based on a comparison between a first focus point of the first eye and a second focus point of the second eye. This way, a condition of a user resulting in different focus point of both eyes, e.g., a fatigue, can be determined in an improved way.

In an example, the condition of the user may be determined based on a comparison between a movement of the first eye and a movement of the second eye. This way, a condition of a user resulting in different movement of both eyes, e.g., a fatigue, can be determined in an improved way.

In an example, the output signal may comprise information indicative of a status of an eye of the user viewing the display during a change of brightness of the display. Further, the processing circuitry may be configured to determine the condition of the user based on an adaption of an eye of the user to the change of the brightness of the display. This way, a further parameter such like an adaption speed of the eye of the user can be used for determining the condition of the user.

In an example, the surgical imaging system may be a microscope system.

In an example, the condition of the user may correspond to a fatigue of the user. This way, a dangerous condition of a user for performing a certain process, e.g., an operation, can be determined.

In an example, the processing circuitry may be further configured to generate a warning signal comprising information indicative of a detected condition (e.g., a fatigue) of the user. Further, the interface circuitry may be further configured to transmit the warning signal to an output device. This way, the user can be informed about the determined condition of the user, e.g., using the display.

Examples provide a display device for an imaging system comprising an eye tracking sensor and interface circuitry configured to transmit a measurement signal comprising information indicative of an output signal of the eye tracking sensor to a device as described above. This way, the display device can provide a measurement of the eye of the user with an integrated eye tracking sensor.

In an example, the display device may further comprise a second eye tracking sensor. The display device may comprise two partially separated direct observation paths. Further, the eye tracking sensor may be integrated into a first direct observation path and the second eye tracking sensor may be integrated into a second direct observation path. This way, both eye tracking sensors can be integrated in an improved way into the display device, e.g., a binocular.

In an example, the display device may be at least one of an ocular, a binocular, a head-mounted display or a monitor.

In an example, the interface circuitry may be configured to receive a signal comprising information indicative of a detected condition of the user from the device. Further, the display device may comprise a processing circuitry configured to output to the user an information to inform the user about the detected condition of the user. This way, the user can be informed in an eased way about a detected condition.

Examples provide a surgical imaging system, comprising a device as described above, a display device and an illumination system.

In an example, the output signal may comprise information indicative of a status of an eye of the user viewing the display during a change of illumination caused by the illumination system. Further, the processing circuitry may be configured to determine the condition of the user based on an adaption of an eye of the user to the change of the illumination. This way, a condition of the user can be determined in an improved way by measuring a reaction of the user to a change of the illumination, resulting in a change of the display brightness.

In an example, the display device may be a display device as described above.

Examples provide a method for a surgical imaging system comprising receiving, from an eye tracking sensor, information about a user viewing a display of a display device and determining a condition of the user based on the obtained information.

Examples further relate to a computer program having a program code for performing the method described above, when the computer program is executed on a computer, a processor, or a programmable hardware component.

### Short description of the Figures

Some examples of apparatuses and/or methods will be described in the following by way of example only, and with reference to the accompanying figures, in which
Fig. la, 1b show a block diagram of an example of a device and a surgical imaging system;
Fig. 2 shows an example of a block diagram of a display device;
Fig. 3 shows an example of method; and
Fig. 4 shows a schematic illustration of a system.

### Detailed Description

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Various examples will now be described more fully with reference to the accompanying drawings in which some examples are illustrated. In the figures, the thicknesses of lines, layers and/or regions may be exaggerated for clarity.

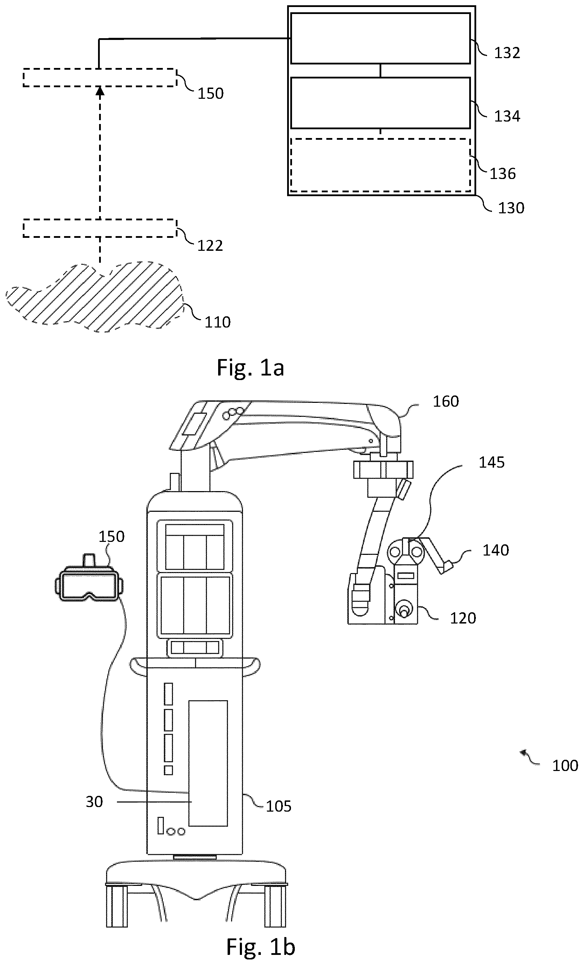

Figs. 1a shows a schematic diagram of an example of a device 130 for a surgical system. The device 130 is tasked with controlling various aspects of a microscope of a surgical system (shown in Fig. 1b), which may be a microscope system and/or with processing various types of sensor data of the surgical system. Consequently, the device 130 may be implemented as a computer system, which interfaces with various components of the surgical system.

The device 130 comprises, as shown in Fig. 1a, interface circuitry 132 and processing circuitry 134. Optionally, the device 130 may further comprise a storage device 136. The processing circuitry 134 is (communicatively) coupled to the one or more interface 132 and to the optional storage device 136. In general, the functionality of the device 130 is provided by the processing circuitry 134, in conjunction with the interface circuitry 132 (for exchanging information, e.g., with at least one optical imaging sensor 122 of a microscope, a display device 150, e.g., of a surgical system (see Fig. 1b), an eye tracking sensor) and/or the optional storage device 136 (for storing and/or retrieving information).

The interface circuitry 132 is configured to receive, from an eye tracking sensor, an output signal comprising information indicative of a status of an eye of a user viewing a display. The processing circuitry 134 is configured to determine a condition of the user based on the received output signal. Thus, the condition of the user working with a surgical system, e.g., a fatigue of the user, can be determined in an improved way. This way, a user of a surgical system can be monitored during the use of the surgical system, e.g., to prevent a failure during an operation utilizing a surgeon surgical system.

The proposed concept is built around two main components - a surgical imaging system, e.g. a surgical microscope equipped with a (set of) camera(s), which comprises the optical components, and which houses a display device 150 being used to view the sample 110, and the device 130, which is used to control the surgical system comprising the microscope, process sensor data of the surgical system, e.g., of the sensor 122, to generate a display signal for the display device 150 of the surgical system and to determine a condition of the user based on the obtained information.

In general, a microscope comprised by the surgical system, is an optical instrument that is suitable for examining objects that are too small to be examined by the human eye (alone). For example, a microscope may provide an optical magnification of a sample, such as a sample 110 shown in Fig. 1a. In modern microscopes, the optical magnification is often provided for a camera or an imaging sensor, such as the optical imaging sensor 122. The microscope may further comprise one or more optical magnification components that are used to magnify a view of the sample, such as an objective (e.g., lens).

There are a variety of different types of microscopes. If the microscope is used in the medical or biological fields, the sample 110 being viewed through the microscope may be a sample of organic tissue, e.g., arranged within a petri dish or present in a part of a body of a patient. In some examples of the present disclosure, e.g., as shown in Fig. 1b, the microscope may be a microscope of a surgical system, i.e., a microscope that is to be used during a surgical procedure, such as an oncological surgical procedure or during tumor surgery. Accordingly, the object being viewed through the microscope, and shown in the image data, may be a sample of organic tissue of a patient, and may be in particular be the surgical site that the surgeon operates on during the surgical procedure. However, the proposed concept may also be applied to other types of surgical imaging system, e.g., microscopy in a laboratory or microscopy for the purpose of material inspection.

Fig. 1b shows a schematic diagram of an example of a surgical system 100 comprising a microscope 120, the device 130, an illumination system (which is used to illuminate an object being imaged by the microscope, e.g., the sample as described above) and a display device, e.g., a digital ocular 140, 145, a monitor or a head-mounted display 152. In general, a (surgical) microscope system is a system that comprises the microscope 120 and additional components, which are operated together with the microscope 120, such as the device 130, the illumination system and the display device.

The surgical system 100 shown in Fig. 1b comprises a number of optional components, such as a base unit 105 (comprising the device 130) with a stand, a head-mounted display 152 and a (robotic or manual) arm 160 which holds the microscope 120 in place, and which is coupled to the base unit 105 and to the microscope 120. In general, these optional and non-optional components may be coupled to the device 130, which may be configured to control and/or interact with the respective components.

While the proposed concept can be used with microscopes that support a fully optical view of the sample through a primary pair of oculars (e.g., as an alternative), the following examples assume that a digital view on a sample is provided. Various examples of the present disclosure thus relate to a surgical system 100 with a digital microscope, i.e., a microscope that uses imaging sensor data generated by at least one optical imaging sensor to generate a digital view of the sample, and to generate a display signal to provide the digital view to the digital viewer or viewers (e.g., the digital ocular 140, 145, a monitor or the head-mounted display 152). In particular, the device 130 may be configured to obtain imaging sensor data from at least one optical imaging sensor of the microscope 120. The device 130 may be configured to provide the digital view of the sample, and to generate a display signal or multiple display signals for the display device 152 of the surgical system 100 to show the digital view of the sample.

In Fig. 1b, the surgical system 100 is shown with three display devices - a primary pair of digital (or optical) oculars 140, which are used by the main surgeon, a secondary pair of digital oculars 145, which are to be used by the assistant that is positioned to the side of the microscope, and a head-mounted display 152, which may be virtual-, augmented- or mixed-reality reality goggles, which may also be used by the main surgeon or by the assistant (or another assistant). For example, the head-mounted display 152 may be a display device that is to be worn by a person, e.g., the main surgeon or an assistant, on or around the head, such that one or two displays are arranged in front of the eye or eyes of the person.

As shown in Fig. 1a the device 130 for a surgical imaging system comprises interface circuitry 132 configured to receive, from an eye tracking sensor, an output signal (e.g., a first output signal) comprising information indicative of a status of an eye of a user viewing a display (e.g., of a display device 150) and processing circuitry 134 configured to determine a condition of the user based on the received output signal. This way, a condition, such like a potential fatigue, of the user, e.g., an operating surgeon, can be determined. For example, by using the output signal from an eye tracking sensor a warning signal can be generated by the processing circuitry 134 and a user can be warned or informed about an own condition, e.g., by use of the display.

Thus, by determining the condition of the user a new functionality may be added to the surgical imaging system. For example, a fatigue of the user may be determined. The output signal of the eye tracking sensor may comprise information indicative of at least one of an eye open or closed, a blink rate, a blink duration, a pupil dilation/contraction or pupil response time or a reaction to changing light condition.

A user condition can be a physical condition of the user, e.g. fatigue, stress, increased brain power.

A fatigue detection can be performed by several approaches. For example, fatigue, e.g., eye fatigue, can be determined based on the blink rate of the user. Blinking gets slower as a person becomes
more tired and can be an indicator of visual fatigue. Blink rate can be captured by an eye tracking sensor, e.g., a camera. Eyes at rest blink about 15 times per minute. Sustained visual tasks can reduce a blink rate to five blinks per minute. The user can be notified to take a break if blink rate falls below 10, 8, 6, 5, and/or 4 blinks per minute. The user could also be alerted to an anomalous blink rate. Blink completeness (opening eyelids completely after a blink) can also be monitored, as well as openness of eyelids (e.g., to detect drowsiness) and squinting. Optionally or alternatively a movement of at least one eye may be evaluated in order to draw conclusions as to the fatigue of the user. The movement of the eye can be distinguished in general at least as a fixation portion and a saccadic portion. The saccadic portion is made up of a rapid eye movement called a "saccade." The saccades can be evaluated. A saccade occurs, for example, in order to switch between two objects being viewed. A slow eye movement, on the other hand, occurs, for example, when a moving object is being tracked.

A correlation exists between a movement velocity of the eye during a saccade and a magnitude of the saccade. The "magnitude" can be understood here as an angle between a beginning of the saccade and an end of the saccade. For a person who is not fatigued, this correlation is reproducible with little variance. As the person becomes fatigued, the variance becomes greater.

Thus, a recognition of fatigue can be based on the eye movement following steps: ascertaining a first saccade and at least one further saccade of an eye movement of a person, using a gaze direction signal that models the eye movement; determining a first data point representing a first amplitude of the first saccade and a first maximum velocity of the first saccade, and at least one further data point representing a further amplitude of the further saccade and a further maximum velocity of the further saccade, using the gaze direction signal and comparing the first data point and at least the further data point with a saccade model, the person being recognized as fatigued if the data points have a predetermined relationship to a confidence region of the saccade model.

A "saccade" can be understood as a movement segment of an eye. The saccade is part of a saccadic period. The saccadic period can be flanked by fixation periods. In the saccade, the eye is moved rapidly from a first angular position into a second angular position. The angle between the first angular position and the second angular position can be referred to as an "amplitude." A maximum angular velocity attained during the saccade can be referred to as a "maximum velocity." A value of the amplitude and a value of the maximum velocity constitute a value pair or a data point. A saccade model numerically models the correlation between the amplitude and the maximum velocity. A confidence region can be referred to as a "tolerance range." The confidence region is delimited by one or two confidence limits. The confidence region can represent a range in a data space, the data space being spanned by the dimensions of the data points. For example, the data space can be spanned in the dimensions of the maximum velocity and the amplitude of the eye movement.

Further, a step of personalizing the saccade model to the user can be performed. Here at least one parameter of the saccade model can be determined using temporally previous (e.g., temporally preceding) data points. Temporally previous or temporally preceding data points that are associated with the user can be used here. The saccade model can be personalized when the person is rested or not fatigued. During personalization, an individual correlation between the amplitude and maximum velocity can be modeled. Personalization allows fatigue affecting the person to be reliably recognized. Alternatively, a standard setup can be used as default configuration, e.g., for a person without given previous data points for individualization.

For example, the saccade model (or a blink rate model) can be personalized using a predetermined minimum number of data points. With a sufficient data inventory, outliers can be recognized and discarded for personalization. For example, the saccade model can be personalized when 100 data points are collected. Data points from a predefined time window can be used. For example, the saccade model can be personalized in a predefined time period after starting to view the display. After starting to view the display, the user is with high probability alert or not fatigued.

The user can be recognized as fatigued if a predetermined proportion of the data points lie outside the confidence region. When data points are located outside the confidence region, the variance of the saccades is elevated, which indicates fatigue affecting the person.

The data points can be weighted. For example, data points below the confidence region can be given a greater weight than data points above the confidence region. Data points above the confidence region can indicate defective detection. Weighting allows fatigue to be recognized quickly and reliably.

In a determining step, additional data points of the saccades, representing the amplitude of a saccade and a duration of the saccade, can be determined using the gaze direction signal. The comparing step can be performed using the additional data points. The additional data points can be used to back up the recognition.

For example, the device 130 can be used for microsurgery, e.g., including, but not limited to neurosurgery, ophthalmology, maxillofacial surgery. Furthermore, the device 130 can be applied to the field of inspection tasks in industrial or life science applications, e.g., watch repairs or general quality control. For example, the device can be applied to application fields where a user is carrying out a critical task that require high concentration on the user's side, such as observing a process or working with delicate samples, e.g. a clockwork of an expensive watch, where fatigue could challenge the outcome and the cost of a project.

In an example, the interface circuitry 132 may be further configured to receive information about a position of the display. Further, the processing circuitry 134 may be configured to determine a focus point of the eye of the user and to determine the condition of the user based on the focus point of the eye of the user and the position of the display. This way, the condition of the user can be determined in an eased way. For example, if the focus point of the eye of the user differs from the position of the display by a certain difference such that the user did not focus the display (looks at the display), the condition of the user may be non-fully awake, e.g. fatigue. This way, a non-fully awake condition of the user can be determined by simply comparing the focus point of the eye of the user with the display position.

Additionally or alternatively a gaze direction of the user can be determined. For example, based on the gaze direction it can be determined if the user looks into the direction of the display. For example, based on the focus point and the gaze direction a three dimensional focus point of the user can be determined. This way, the determination if the user looks at the display can be improved.

In an example, the information about the user viewing the display may correspond to a first eye of the user. Further, the interface circuitry 132 may be configured to receive, from a further eye tracking sensor, a second output signal comprising information indicative of a status of a second eye of the user viewing the display. Further, the processing circuitry 134 may be configured to determine a condition of the user based on the received (first) output signal and the second output signal for the first eye and the second eye. This way, parameters for both eyes can be considered, e.g., a determination of a status can be performed by comparison between both eyes.

For example, a detection of a user condition, e.g., an operating surgeon, a person carrying out critical tasks while observing or working on a sample 110, by checking whether both eyes of the user are working synchronously can be performed by the processing circuitry 134. If both eyes are not working synchronously the condition of the user may be non-fully awake. Optionally, the processing circuitry 134 can check if both eyes work normally, e.g., with respect to a blink rate or an eye movement as described above. For example, an eye may work normally when it shows a movement in line with those of a fully awake state, e.g., with little or no delay in moving around and looking at the same detail.

A check whether both eyes are working synchronously can be performed in an improved way by use of two separate eye tracking sensors, one eye tracking sensor per eye of the user. Further, an analyzation of the gaze direction/position and a comparison with patterns representing normal and tired condition can be done be the processing circuitry 134. This analysis/ pattern comparison can be implemented either by a classical algorithm or by a trained machine learning algorithm.

The machine-learning model is a data structure and/or set of rules representing a statistical model that the processing circuitry 134 uses to perform the above tasks without using explicit instructions, instead relying on models and inference. The data structure and/or set of rules represents learned knowledge (e.g. based on training performed by a machine-learning algorithm). For example, in machine-learning, instead of a rule-based transformation of data, a transformation of data may be used, that is inferred from an analysis of historical and/or training data. In the proposed technique, the content of eye tracking sensor data is analyzed using the machine-learning model (i.e. a data structure and/or set of rules representing the model).

The machine-learning model is trained by a machine-learning algorithm. The term "machine-learning algorithm" denotes a set of instructions that are used to create, train or use a machine-learning model. For the machine-learning model to analyze the content of eye tracking sensor data, the machine-learning model may be trained using training and/or historical eye tracking sensor data as input and training content information (e.g., a condition of the user) as output. By training the machine-learning model with a large set of training eye tracking sensor data and associated training content information (e.g. labels or annotations), the machine-learning model "learns" to recognize the content of the eye tracking sensor data, so the content of eye tracking sensor data that are not included in the training data can be recognized using the machine-learning model. By training the machine-learning model using training eye tracking sensor data and a desired output, the machine-learning model "learns" a transformation between the eye tracking sensor data and the output, which can be used to provide an output based on non-training eye tracking sensor data provided to the machine-learning model.

The machine-learning model may be trained using training input data (e.g. training eye tracking sensor data). For example, the machine-learning model may be trained using a training method called "supervised learning". In supervised learning, the machine-learning model is trained using a plurality of training samples, wherein each sample may comprise a plurality of input data values, and a plurality of desired output values, i.e., each training sample is associated with a desired output value. By specifying both training samples and desired output values, the machine-learning model "learns" which output value to provide based on an input sample that is similar to the samples provided during the training. For example, a training sample may comprise training eye tracking sensor data as input data and one or more labels as desired output data. The labels indicate the condition of the user (e.g. fatigue, stress).

Apart from supervised learning, semi-supervised learning may be used. In semi-supervised learning, some of the training samples lack a corresponding desired output value. Supervised learning may be based on a supervised learning algorithm (e.g. a classification algorithm or a similarity learning algorithm). Classification algorithms may be used as the desired outputs of the trained machine-learning model are restricted to a limited set of values (categorical variables), i.e., the input is classified to one of the limited set of values (type of exercise, execution quality). Similarity learning algorithms are similar to classification algorithms but are based on learning from examples using a similarity function that measures how similar or related two objects are.

Apart from supervised or semi-supervised learning, unsupervised learning may be used to train the machine-learning model. In unsupervised learning, (only) input data are supplied and an unsupervised learning algorithm is used to find structure in the input data such as training and/or historical eye tracking sensor data (e.g. by grouping or clustering the input data, finding commonalities in the data). Clustering is the assignment of input data comprising a plurality of input values into subsets (clusters) so that input values within the same cluster are similar according to one or more (predefined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such that the cumulative reward is increased, leading to software agents that become better at the task they are given (as evidenced by increasing rewards).

Furthermore, additional techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input eye tracking sensor data) may be represented by the branches of the decision tree, and an output value corresponding to the item may be represented by the leaves of the decision tree. Decision trees support discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may, e.g., be used to store, manipulate or apply the knowledge.

For example, the machine-learning model may be an Artificial Neural Network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receive input values (e.g. the eye tracking sensor data), hidden nodes that are (only) connected to other nodes, and output nodes that provide output values (e.g. type and/or execution quality of the physical exercise). Each node may represent an artificial neuron. Each edge may transmit information from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an ANN may comprise adjusting the weights of the nodes and/or edges of the ANN, i.e., to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values (e.g. eye tracking sensor data) that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection. In some examples, the machine-learning model may be a combination of the above examples.

In an example, the condition of the user may be determined based on a comparison between a first focus point of the first eye and a second focus point of the second eye. In an example, the condition of the user may be determined based on a comparison between a movement of the first eye and a movement of the second eye. By comparing the focus points/movements of both eyes of the user a fatigue of the user can be determine even if only one eye shows fatigue. Further, resource intensive training of an ML algorithm and/or an effort for personalization can be reduced since the condition of the user can be determined by a simplified comparison of the focus points. Thus, a method with less computational effort can be provided.

In an example, the output signal may comprise information indicative of a status of an eye of the user viewing the display during a change of brightness of the display. Further, the processing circuitry 134 may be configured to determine the condition of the user based on an adaption of an eye of the user to the change of the brightness of the display. This way, the determination of the user condition can be triggered. For example, the processing circuitry 134 may be configured to generate a control signal for the display. Further, the interface circuitry 132 may be configured to transmit the control signal to the display. This way, the device 130 can trigger a time when a determination of the condition of user can be performed, e.g., with a periodically frequency, if a non-fully awake condition was determined (e.g., to double-check the determination of the condition). For example, if an adaption of the eye of the user (e.g., an adaption of a pupil diameter, a blink rate, an eye closure time) to the change of the brightness is not observed in a certain time limit by the eye tracking sensor, the user may be in a non-fully awake condition.

In an example, the surgical imaging system may be a (surgical) microscope system. In an example, the condition of the user may correspond to a fatigue of the user.

In an example, the processing circuitry 134 may be further configured to generate a warning signal comprising information indicative of a detected condition (e.g., a fatigue) of the user. Further, the interface circuitry 132 may be further configured to transmit the warning signal to an output device. For example, the output device may be the display. The display may display an information to the user about the condition of the user. Alternatively or optionally the output signal may be a loudspeaker or a device which can vibrate to give haptic feedback.

The device 130 can be combined with different displays, e.g., with binoculars, head-mounted display, a (three-dimensional) monitor.

As shown in Fig. 1a the respective interface circuitry 132 is coupled to the respective processing circuitry 134 at the device 130. In examples the processing circuitry 134 may be implemented using one or more processing units, one or more processing devices, any means for processing, such as a processor, a computer or a programmable hardware component being operable with accordingly adapted software. Similar, the described functions of the processing circuitry 134 may as well be implemented in software, which is then executed on one or more programmable hardware components. Such hardware components may comprise a general-purpose processor, a Digital Signal Processor (DSP), a micro-controller, etc. The processing circuitry 134 is capable of controlling the interface circuitry 132, so that any data transfer that occurs over the interface circuitry 132 and/or any interaction in which the interface circuitry 132 may be involved may be controlled by the processing circuitry 134.

In an embodiment the device 130 may comprise a storage device 136 and at least one processing circuitry 134 operably coupled to the storage device 136 and configured to perform the below mentioned method.

In examples the interface circuitry 132 may correspond to any means for obtaining, receiving, transmitting or providing analog or digital signals or information, e.g. any connector, contact, pin, register, input port, output port, conductor, lane, etc. which allows providing or obtaining a signal or information. The interface circuitry 132 may be wireless or wireline and it may be configured to communicate, e.g., transmit or receive signals, information with further internal or external components.

The device 130 may be a computer, processor, control unit, (field) programmable logic array ((F)PLA), (field) programmable gate array ((F)PGA), graphics processor unit (GPU), application-specific integrated circuit (ASICs), integrated circuits (IC) or system-on-a-chip (SoCs) system.

As shown in Fig. 1b the surgical imaging system may comprise a device 130, a display device 140, 145, 152 and an illumination system (not shown). For example, the display device 140, 145, 152 may comprise a display that is viewed by the user and an interface circuitry to communicate with the device 130.

In an example, the output signal may comprise information indicative of a status of an eye of the user viewing the display during a change of illumination caused by the illumination system. Further, the processing circuitry may be configured to determine the condition of the user based on an adaption of an eye of the user to the change of the illumination. For example, the illumination system can be used to vary an illumination of a sample. For example, the sample can be illuminated by the illumination system to cause fluorescence or merely by white light. Thus, an image of the sample displayed in a display of the display device 140, 145, 152 could vary in a brightness caused by the changed illumination of the surgical imaging system 100. This way, the illumination system of the surgical imaging system 100 can be used to trigger a determination of the condition of the user. For example, the processing circuitry can generate a control signal for the illumination system and thus can trigger a time when a determination of the condition of user can be performed as described above. For example, the processing circuitry can generate a control signal to switch the illumination system from an imaging mode to a mode with a different overall light intensity.

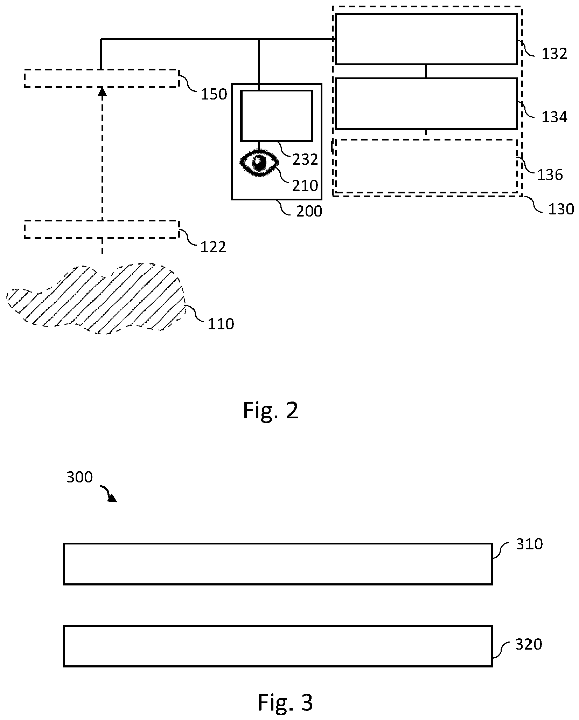

More details and aspects are mentioned in connection with the examples described below. The example shown in Fig. 1 may comprise one or more optional additional features corresponding to one or more aspects mentioned in connection with the proposed concept or one or more examples described below (e.g., Fig. 2 - 4).

Fig. 2 shows an example of a block diagram of a display device 200. The display device 200 for a surgical imaging system comprises an eye tracking sensor 210 and interface circuitry 232 configured to transmit a measurement signal comprising information indicative of an output signal of the eye tracking sensor to a device as described with reference to Fig. 1. For example, the display device 200 may provide the (first) output signal and/or the second output signal for the device as described with reference to Fig. 1. This way, no further effort is needed to integrate an eye tracking sensor into the surgical imaging system.

In an example, the display device 200 may further comprise a second eye tracking sensor. The display device 200 may comprise two partially separated direct observation paths. Further, the eye tracking sensor may be integrated into a first direct observation path and the second eye tracking sensor may be integrated into a second direct observation path. Thus, for each of the user a specific eye tracking sensor can be integrated into the display device 200. For example, if the display device is a binocular, an eye tracking sensor can be integrated into each ocular of the binocular.

In an example, the display device may be at least one of an ocular, a binocular, a head-mounted display or a (three-dimensional) monitor.

In an example, the interface circuitry 232 may be configured to receive a signal comprising information indicative of a detected condition of the user from the device. Further, the display device may comprise a processing circuitry configured to output to the user an information to inform the user about the detected condition of the user. This way, the user can be informed by the display of the display about a detected condition of its own, e.g., a fatigue.

More details and aspects are mentioned in connection with the examples described above and/or below. The example shown in Fig. 2 may comprise one or more optional additional features corresponding to one or more aspects mentioned in connection with the proposed concept or one or more examples described above (e.g., Fig. 1) and/or below (e.g., Fig. 3-4).

Fig. 3 shows an example of method 300. The method 300 comprises receiving 310, from an eye tracking sensor, an output signal comprising information indicative of a status of an eye of a user viewing a display and determining 320 a condition of the user based on the received output signal. The method 300 may be performed by the device as described with reference to Fig. 1. This way, user condition can be determined in an improved way for a surgical imaging system.

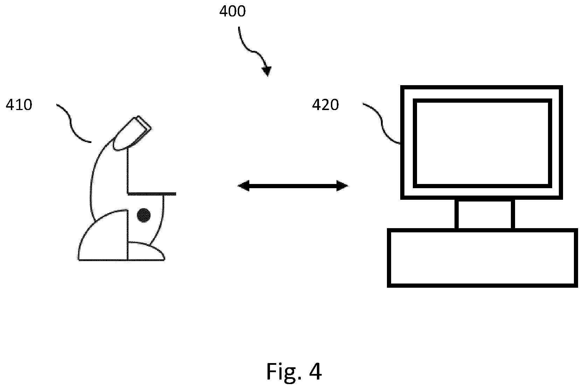

More details and aspects are mentioned in connection with the examples described above and/or below. The example shown in Fig. 3 may comprise one or more optional additional features corresponding to one or more aspects mentioned in connection with the proposed concept or one or more examples described above (e.g., Fig. 1-2) and/or below (e.g., Fig. 4).

Fig. 4 shows a schematic illustration of a system 400 configured to perform a method described herein. The system 400 comprises a microscope 410 and a computer system 420. The microscope 410 is configured to take images and is connected to the computer system 420. The computer system 420 is configured to execute at least a part of a method described herein. The computer system 420 may be configured to execute a machine learning algorithm. The computer system 420 and microscope 410 may be separate entities but can also be integrated together in one common housing. The computer system 420 may be part of a central processing system of the microscope 410 and/or the computer system 420 may be part of a subcomponent of the microscope 410, such as a sensor, an actor, a camera or an illumination unit, etc. of the microscope 410.

The computer system 420 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 420 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

More details and aspects are mentioned in connection with the examples described above. The example shown in Fig. 4 may comprise one or more optional additional features corresponding to one or more aspects mentioned in connection with the proposed concept or one or more examples described above (e.g., Fig. 1 - 3).

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable. Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### List of reference Signs

100 surgical imaging system
105 base unit
110 sample
120 microscope
122 imaging sensor
130 device
132 interface circuitry
134 processing circuitry
136 storage device
140, 145 digital ocular
150 display device
152 head-mounted display
160 arm
200 display device
210 eye tracking sensor
232 interface circuitry
300 method for a surgical imaging system
310 receiving an output signal
320 determining a condition of a user
400 system
410 microscope
420 computer system

## Claims

1. A device (130) for a surgical imaging system, comprising:
interface circuitry (132) configured to receive, from an eye tracking sensor, an output signal comprising information indicative of a status of an eye of a user viewing a display; and
processing circuitry (134) configured to determine a condition of the user based on the received output signal.

2. The device (130) according to claim 1, wherein
the interface circuitry (132) is further configured to receive information about a position of the display, and wherein
the processing circuitry (134) is further configured to:
determine a focus point of the eye of the user; and
determine the condition of the user based on the focus point of the eye of the user and the position of the display.

3. The device (130) according to any one of the preceding claims, wherein the information about the user viewing the display corresponds to a first eye of the user, and wherein
the interface circuitry (132) is further configured to receive, from a further eye tracking sensor, a second output signal comprising information indicative of a status of a second eye of the user viewing the display; and wherein
the processing circuitry (134) is further configured to determine a condition of the user based on the received output signal and the second output signal for the first eye and the second eye.

4. The device (130) according to claim 3, wherein
the condition of the user is determined based on a comparison between a first focus point of the first eye and a second focus point of the second eye.

5. The device (130) according to claim 3 or 4, wherein
the condition of the user is determined based on a comparison between a movement of the first eye and a movement of the second eye.

6. The device (130) according to any one of the preceding claims, wherein
the condition of the user is determined based on a distance between a focal plane of an eye of the user and an image plane of the display.

7. The device (130) according to any one of the preceding claims, wherein
the output signal comprises information indicative of a status of an eye of the user viewing the display during a change of brightness of the display; and wherein
the processing circuitry (134) is further configured to determine the condition of the user based on an adaption of an eye of the user to the change of the brightness of the display.

8. The device (130) according to claim 7, the processing circuitry (134) is further configured to generate a warning signal comprising information indicative of a detected condition of the user; and wherein
the interface circuitry (132) is further configured to transmit the warning signal to an output device.

9. A display device (200) for a surgical imaging system, comprising:
an eye tracking sensor (210); and
interface circuitry (232) configured to transmit a measurement signal comprising information indicative of an output signal of the eye tracking sensor to a device (130) according to any of the preceding claims.

10. The display device (200) according to claim 9, further comprising
a second eye tracking sensor, wherein the display device (200) comprises two partially separated direct observation paths; and wherein
the eye tracking sensor is integrated into a first direct observation path and the second eye tracking sensor is integrated into a second direct observation path.

11. The display device (200) according to claim 9 or 10, wherein
the display device is at least one of an ocular, a binocular (140; 145), a head-mounted display (152) or a monitor.

12. The display device (200) according to any one of the claims 9 - 11, wherein the interface circuitry (232) is configured to
receive a signal comprising information indicative of a detected condition of the user from the device (130); and
further comprising a processing circuitry configured to output to the user an information to inform the user about the detected condition of the user.

13. A surgical imaging system (100), comprising:
a device (130) according to any one of the claims 1-8;
a display device (150; 140; 145; 152); and
an illumination system.

14. A method (300) for a surgical imaging system, comprising:
receiving (310), from an eye tracking sensor, an output signal comprising information indicative of a status of an eye of a user viewing a display; and
determining (320) a condition of the user based on the received output signal.

15. A computer program having a program code for performing a method according to claim 14 when the program is executed on a processor.
